# EUROPÄISCHE PATENTSCHRIFT

(11) **EP 3 332 705 B1**
(45) Veröffentlichungstag und Bekanntmachung des Hinweises auf die Patenterteilung: **18.09.2019**
(21) Anmeldenummer: 16203387.2
(22) Anmeldetag: 12.12.2016
(51) Int. Cl.: A61B 5/055

(54) **VERFAHREN ZUR ERFASSUNG EINES DENTALEN OBJEKTS**
METHOD FOR DETECTING A DENTAL OBJECT
PROCÉDÉ DE DÉTECTION D'UN OBJET DENTAIRE

(43) Veröffentlichungstag der Anmeldung: 13.06.2018
(73) Patentinhaber: Sirona Dental Systems GmbH, 64625 Bensheim (DE)
(72) Erfinder: ABKAI, Ciamak, 68542 Heddesheim (DE); BRAUN, Tim, 64521 Groß-Gerau (DE)
(74) Vertreter: Özer, Alpdeniz

(56) Entgegenhaltungen:
- ELEY KAREN A ET AL: "Black Bone MRI: a potential alternative to CT with three-dimensional reconstruction of the craniofacial skeleton in the diagnosis of craniosy", EUROPEAN RADIOLOGY, SPRINGER INTERNATIONAL, BERLIN, DE, Bd. 24, Nr. 10, 20. Juli 2014 (2014-07-20) , Seiten 2417-2426, XP035385411, ISSN: 0938-7994, DOI: 10.1007/S00330-014-3286-7 [gefunden am 2014-07-20]

## Beschreibung

### Technisches Gebiet

Die Erfindung betrifft ein Verfahren zur Erfassung eines dentalen Objekts eines Patienten mit einem Objektvolumen, insbesondere zumindest eines Teils eines Schädels, eines Oberkiefers und/oder eines Unterkiefers.

### Stand der Technik

Aus dem Stand der Technik sind mehrere Verfahren zur Erstellung von MRT-Aufnahmen von dentalen Objekten, insbesondere zumindest eines Teils eines Schädels, bekannt.

Nach einem bekannten Verfahren werden mittels einer herkömmlichen MRT-Vorrichtung einzelne MRT-Schnittaufnahmen durchgeführt, die beispielsweise eine Midsagittalebene oder einen Teil eines Oberkiefers bzw. eines Unterkiefers beinhalten. Die einzelne MRT-Schnittaufnahme bildet dann eine festgelegte Schnittebene durch den Schädel des Patienten ab.

Ein Nachteil dieses Verfahrens besteht darin, dass die manuelle Planung der einzelnen MRT-Schnittaufnahmen mit erheblichem Zeitaufwand verbunden ist. Die einzelnen MRT-Schnittaufnahmen sind nicht zusammenhängend und erschweren damit die Diagnose.

Bei einem weiteren Verfahren können mehrere zueinander parallel angeordnete MRT-Schnittaufnahmen aufgenommen werden, so dass aus den einzelnen MRT-Schnittaufnahmen eine dreidimensionale MRT-Gesamtaufnahme des gesamten Schädels erzeugt werden kann.

Ein Nachteil dieses Verfahrens besteht darin, dass die Vermessung der einzelnen MRT-Schnittaufnahmen eine lange Vermessungsdauer in Anspruch nimmt. Ein weiterer Nachteil dieses Verfahrens ist, dass der gesamte Schädel mit allen darin enthaltenen anatomischen Strukturen vermessen wird und die Diagnose bestimmter Strukturen im Schädel des Patienten erschwert werden kann.

Ein Verfahren gemäss der Präambel von Anspruch 1 wird von folgender Veröffentlichung offenbart:
ELEY KAREN A ET AL: "Black Bone MRI: a potential alternative to CT with three-dimensional reconstruction of the craniofacial skeleton in the diagnosis of craniosy",EUROPEAN RADIOLOGY, Bd. 24, Nr. 10, 20. Juli 2014, Seiten 2417-2426.

Die Aufgabe der vorliegenden Erfindung besteht daher darin ein Verfahren zur Erfassung eines dentalen Objekts bereitzustellen, bei dem die Aufnahmedauer verkürzt wird und die Bildqualität bestimmter aufzunehmender Strukturen verbessert wird.

### Darstellung der Erfindung

Die Erfindung betrifft ein Verfahren zur Erfassung eines dentalen Objekts eines Patienten mit einem Objektvolumen, insbesondere zumindest eines Teils eines Schädels, eines Oberkiefers und/oder eines Unterkiefers. Zur Durchführung des Verfahrens werden mehrere Segment-Volumenbereiche im Objektvolumen festgelegt, wobei die Segment-Volumenbereiche sich höchstens teilweise überlagern und Vorzugsrichtungen der Segment-Volumenbereiche nicht parallel zueinander sind, wobei Bilddaten der Segment-Volumenbereiche mittels einer MRT-Vorrichtung innerhalb eines Meßvolumens der MRT-Vorrichtung aufgenommen werden, wobei aus den Bilddaten der einzelnen Segment-Volumenbereiche computergestützt ein zweidimensionales Kompositbild durch Projektion der Bilddaten auf eine Zielebene erzeugt wird, wobei die Zielebene parallel zu einer festgelegten Midsagittalebene des Schädels angeordnet ist oder der Midsagittalebene entspricht, wobei ein erster Segment-Volumenbereich zumindest teilweise die Midsagittalebene des Schädels umfasst.

Das dentale Objekt kann zumindest ein Teil eines Schädels, ein Unterkiefer und/oder ein Oberkiefer sein. Das Objekt kann auch bestimmte anatomische Strukturen, wie die Kiefergelenke, den Kieferkammbogen, das Kinn, vordere Schädelbasis und/oder Unterkieferrand, enthalten.

Die MRT-Vorrichtung (Magnetresonanztomographie-Vorrichtung) ist eine herkömmliche MRT-Vorrichtung zur Vermessung eines Schädels. Die MRT-Vorrichtung weist dabei ein Messvolumen auf, wobei das Objektvolumen des Objekts das innerhalb des Messvolumens angeordnet wird, um das Objekt zu vermessen. Nach dem vorliegenden Verfahren werden mehrere Segment-Volumenbereiche im Objektvolumen festgelegt.

Die Segment-Volumenbereiche können beliebig geformt sein und beispielsweise eine quaderförmige Geometrie aufweisen. Die Segment-Volumenbereiche können auch eine gekrümmte Form aufweisen, wobei eine besondere MRT-Vorrichtung verwendet wird, die einen solchen gekrümmten Segment-Volumenbereich aufnehmen kann. Bei einer solchen MRT-Vorrichtung können beispielsweise die Gradientenspulen so angeordnet sein, dass die Isolinien eines Gradientenfeldes gekrümmt verlaufen.

Die Bilddaten der Segment-Volumenbereiche werden dann mittels der MRT-Vorrichtung aufgenommen. Bei einer ersten Alternative werden die Bilddaten der Segment-Volumenbereiche aufgenommen, in dem eine einzelne dreidimensionale MRT-Gesamtaufnahme des gesamten Objekts aufgenommen wird und die Bilddaten der einzelnen Segment-Volumenbereiche aus der dreidimensionalen MRT-Gesamtaufnahme ausgeschnitten und übernommen werden. Bei einer zweiten Alternative werden die Bilddaten des Segment-Volumenbereichs aufgenommen, in dem mehrere MRT-Segmentaufnahmen, also mindestens zwei MRT-Segmentaufnahmen mittels der MRT-Vorrichtung aufgenommen werden, die die jeweiligen festgelegten Segment-Volumenbereiche abbilden. Diese Alternative hat den Vorteil, dass die Dauer der gesamten Vermessung verkürzt wird, da nur die Segment-Volumenbereiche einzeln nacheinander vermessen werden.

Die Segment-Volumenbereiche sind dabei so festgelegt, dass sie sich höchstens teilweise überlappen. Die Segment-Volumenbereiche beinhalten sich also nicht vollständig.

Eine Vorzugsrichtung eines quaderförmigen Segment-Volumenbereichs kann beispielsweise durch die längste Seitenkante definiert sein. Bei einem beliebig geformten Segment-Volumenbereich kann die Vorzugsrichtung auch als der größte Innendurchmesser dieses Segment-Volumenbereichs definiert sein.

Der erste Segment-Volumenbereich umfasst zumindest teilweise die Midsagittalebene des Schädels, so dass insbesondere wesentliche anatomische Strukturen, wie die Schädelbasis, das Kinn und/oder die Frontzähne, darin abgebildet werden.

Die Midsagittalebene ist eine sagittale Ebene (also eine Ebene, welche bei einer senkrechten Blickrichtung eine seitliche Ansicht des Körpers zeigt), welche den Patientenkopf in zwei symmetrische Hälften aufteilt.

Aus den Bilddaten der einzelnen Segment-Volumenbereiche wird computergestützt das zweidimensionale Kompositbild durch Projektion der Bilddaten auf die Zielebene erzeugt, die parallel zu der festgelegten Midsagittalebene des Schädels angeordnet ist oder der Midsagittalebene entspricht.

Die Bilddaten der einzelnen Segment-Volumenbereiche können auch mit festgelegten Gewichtungsfaktoren gewichtet werden und auf die Zielebene projiziert werden. Die Gewichtungsfaktoren können beispielsweise von der Abgrenzung bzw. Kontrast der anatomischen Strukturen in den einzelnen Segment-Volumenbereichen abhängen. Die Gewichtungsfaktoren können dabei dynamisch in Abhängigkeit von den anatomischen Strukturen berechnet werden oder auch statisch festgelegt sein. Durch die unterschiedliche Gewichtung der einzelnen Segment-Volumenbereiche wird ermöglicht, dass die anatomischen Strukturen der verschiedenen Segment-Volumenbereiche in Überlagerung im Kompositbild deutlich mit einem ausreichenden Kontrast zueinander dargestellt werden. Die Gewichtungsfaktoren können beispielsweise einen Wert von 0,5 aufweisen.

Dadurch wird also ein Kompositbild erzeugt, dass einer herkömmlichen zephalometrischen Röntgenaufnahme (Fernröntgenaufnahme) entspricht bzw. nachgebildet ist und insbesondere zur zephalometrischen Analyse verwendet werden kann. Durch die parallele Projektion der Bilddaten auf die Zielfläche sind die wesentlichen Abstände zwischen definierten anatomischen Strukturen, wie der vorderen Schädelbasis, dem Kinn, den Kiefergelenken, dem Unterkiefer und/oder den Oberkiefer, in zephalometrischen Kompositbild nicht verzerrt und können präzise ausgemessen werden. Die zephalometrische Diagnose wird insbesondere verwendet, um eine Okklusionsebene, eine Bisshöhe und/oder eine Okklusionskurve zu bestimmen. Die ermittelten Messgrößen können dann in der Prothetik zur Planung einer Rekonstruktion von Abrasionsfällen oder zur Planung von Vollprothesen aus mehreren Zähnen verwendet werden. Denn die zephalometrische Diagnose gibt wertvolle Hinweise zur Positionierung der einzelnen Zähne und der Kiefer relativ zum Schädel und insbesondere zum Kiefergelenk.

Ein Vorteil des vorliegenden Verfahrens besteht darin, dass nicht der gesamte Schädel vermessen wird, sondern festgelegte Segment-Volumenbereiche definiert werden, die die relevanten anatomischen Strukturen, wie den Oberkiefer, den Unterkiefer, die vordere Schädelbasis, das Kinn und/oder die Kiefergelenke enthalten. Das erzeugte zweidimensionale Kompositbild, das einem herkömmlichen zephalometrischen Röntgenbild entspricht, enthält also lediglich die Projektion der relevanten Segment-Volumenbereiche, so dass die übrigen Zwischenbereiche des Schädels ausgeblendet werden. Dadurch werden die Diagnose und die Bestimmung der relevanten anatomischen Strukturen erleichtert.

Bei einer herkömmlichen MRT-Vorrichtung erfolgt eine Ortskodierung der Voxel des Meßvolumens indem die ausgelesenen Signale den einzelnen Volumenelemente (Voxeln) zugeordnet werden, wobei mit linear ortsabhängigen Magnetfeldern (Gradientenfeldern) eine Ortskodierung erzeugt wird. Dabei wird ausgenutzt, dass für ein bestimmtes Teilchen die Larmorfrequenz von der magnetischen Flussdichte abhängt (je stärker der Feldanteil senkrecht zur Richtung des Teilchendrehimpulses, desto höher die Larmorfrequenz).

Ein erster Gradient liegt bei der Anregung an und stellt sicher, dass nur eine einzelne Schicht des Körpers die passende Larmorfrequenz besitzt, also nur die Spins dieser Schicht ausgelenkt werden (Schichtselektionsgradient).

Ein zweiter Gradient quer zum ersten wird nach der Anregung kurz eingeschaltet und bewirkt eine kontrollierte Dephasierung der Spins dergestalt, dass in jeder Bildzeile die Präzession der Spins eine andere Phasenlage hat (Phasenkodiergradient).

Der dritte Gradient wird während der Messung rechtwinklig zu den beiden anderen geschaltet; er sorgt dafür, dass die Spins jeder Bildspalte eine andere Präzessionsgeschwindigkeit haben, also eine andere Larmorfrequenz senden (Auslesegradient, Frequenzkodiergradient).

Alle drei Gradienten zusammen bewirken also eine Kodierung des Signals in drei Raumebenen. Das empfangene Signal gehört zu einer bestimmten Schicht des Körpers und enthält eine Kombination aus Frequenz- und Phasenkodierung, die der Computer mit einer Fourier-Transformation in ein zweidimensionales Bild umrechnen kann.

Vorteilhafterweise kann ein zweiter Segment-Volumenbereich festgelegt werden, der zumindest einen Teil des Oberkiefers und/oder des Unterkiefers umfasst, wobei aus dem ersten Segment-Volumenbereich und dem zweiten Segment-Volumenbereich durch Projektion auf die Zielebene das zweidimensionale Kompositbild erzeugt wird.

Dadurch wird also der erste Segment-Volumenbereich mit der Midsagittalebene und der zweite Segment-Volumenbereich mit zumindest einem Teil des Oberkiefers und/oder des Unterkiefers auf die Zielebene projiziert, um das zweidimensionale Kompositbild zu erzeugen. Dabei kann beispielsweise eine linke Hälfte des Oberkiefers und/oder des Unterkiefers im zweiten Segment-Volumenbereich enthalten sein. Dies ermöglicht dann eine zephalometrische Diagnose der linken Hälfte des Oberkiefers bzw. des Unterkiefers relativ zu den relevanten anatomischen Strukturen in der Midsagittalebene des ersten Segment-Volumenbereichs. Eine solche Form der Diagnostik ist mittels herkömmlicher zephalometrischer Aufnahmen nicht möglich, da immer Strukturen des linken und rechten Kieferbereiches sich überlagern. Der zweite Segment-Volumenbereich kann auch die rechte Hälfte des Oberkiefers und/oder des Unterkiefers umfassen, so dass die zephalometrische Diagnose der rechten Hälfte ermöglicht wird.

Vorteilhafterweise kann der zweite Segment-Volumenbereich eine linke Seite des Oberkiefers und/oder des Unterkiefers umfassen, wobei zusätzlich ein dritter Segment-Volumenbereich festgelegt wird, der eine rechte Seite des Oberkiefers und/oder des Unterkiefers umfasst, wobei aus dem ersten Segment-Volumenbereich, dem zweiten Segment-Volumenbereich und dem dritten Segment-Volumenbereich durch Projektion auf die Zielebene das zweidimensionale Kompositbild erzeugt wird.

Dadurch werden also die Bilddaten des ersten Segment-Volumenbereichs mit der Midsagittalebene, die Bilddaten des zweiten Segment-Volumenbereichs mit der linken Seite und die Bilddaten des dritten Segment-Volumenbereichs mit der rechten Seite durch Projektion überlagert und das zweidimensionale Kompositbild erzeugt. Idealerweise sollte das linke Kiefergelenk der linken Seite mit dem rechten Kiefergelenk der rechten Seite in Überlagerung gebracht werden, so dass die Achse zwischen den beiden Kiefergelenken senkrecht zur Zielebene angeordnet ist (falls keine bifaciale Asymmetrie vorliegt).

Ein besonderer Vorteil des vorliegenden Verfahrens im Vergleich zur herkömmlichen kephalometrischen Röntgenaufnahme liegt also darin, dass auch Asymmetrien zwischen der linken Seite des Schädels und der rechten Seite des Schädels in Bezug auf die Midsagittalebene dargestellt werden können.

Vorteilhafterweise kann jeder Segment-Volumenbereich bezüglich seiner Lage und Ausrichtung innerhalb des Meßvolumens der MRT-Vorrichtung anhand einer Voreinstellung für den jeweiligen Patienten oder anhand einer Voraufnahme des Objekts festgelegt werden.

Die Positionierung des Patienten relativ zur MRT-Vorrichtung kann beispielsweise mittels einer Positionierungsvorrichtung, wie einer Kopfhalterung und/oder einer Aufbisshalterung, erfolgen. Der erste Segmentvolumenbereich mit der Midsagittalebene ist dann relativ zur MRT-Vorrichtung und relativ zur Positionierungsvorrichtung in seiner Lage und Ausrichtung definiert. In Abhängigkeit von der Größe des Schädels des Patienten sind die beiden Seiten des Oberkiefers und/oder des Unterkiefers weiter weg oder näher an die Midsagittalebene angeordnet. Die Voreinstellung der MRT-Vorrichtung kann dem Patienten beispielsweise ermöglichen, zwischen verschiedenen Programmen, beispielsweise für ein Kinder-Programm oder ein Erwachsenen-Programm auszuwählen. Dabei werden der zweite Segment-Volumenbereich mit der linken Seite des Kiefers und der dritte Segment-Volumenbereich des Kiefers so eingestellt, dass die Kiefer eines Musterkopfes eines Kindes bzw. eines Erwachsenen hineinpassen. Die Voreinstellung kann auch für den jeweiligen Patienten erfolgen, falls der Verlauf und die Anordnung der Kiefer des Patienten vorbekannt sind. Der Verlauf und die Anordnung der Kiefer kann auch aus der Voraufnahme des Patienten ermittelt werden, wobei die Voraufnahme eine zweidimensionale Aufnahme, beispielsweise in einer Tranversalebene, oder eine dreidimensionale Aufnahme sein kann. Die Voraufnahme kann eine Röntgenaufnahme oder eine MRT-Aufnahme sein.

Vorteilhafterweise kann eine Tiefe des Segment-Volumenbereichs zwischen 0,5 mm und 30 mm, vorzugsweise zwischen 1 mm und 15 mm, betragen.

Dadurch ist der Oberkiefer und/oder der Unterkiefer innerhalb des Segment-Volumenbereiches vollständig enthalten und wird demnach vollständig als Projektion im Kompositbild abgebildet.

Vorteilhafterweise kann der Segment-Volumenbereich quaderförmig sein oder eine gekrümmte längliche Form aufweisen.

Die Geometrie des Segment-Volumenbereichs ist durch den Aufbau der MRT-Vorrichtung und insbesondere der Gradientenspulen der MRT-Vorrichtung beeinflusst.

Bei einer herkömmlichen MRT-Vorrichtung werden drei Gradientenspulen verwendet, die lineare Gradientenfelder entlang der x, y, z Achsen einer Patientenöffnung erzeugen. Damit kann durch Überlagerung der Gradientenfelder eine beliebig im Raum drehbare planare bzw. nicht gekrümmte Fläche eine eindeutige Resonanzfrequenz erhalten. Der Anregungsbereich mittels herkömmlicher 1D Pulse ist die angeregte planare Fläche extrudiert entlang der drei Flächenachsen und stellt damit einen beliebig im Raum orientierten Quader dar.

Vorteilhafterweise kann das Kompositbild einer herkömmlichen zephalometrischen Röntgenaufnahme (Fernröntgenaufnahme) entsprechen, wobei die Zielebene des Kompositbildes einer Ebene entspricht, die parallel zu einer Detektorfläche eines Röntgendetektors bei einer zephalometrischen Röntgenaufnahme angeordnet ist.

Dadurch ermöglicht das erzeugte Kompositbild eine zephalometrische Diagnose des Patienten. Das erzeugte Kompositbild hat jedoch im Vergleich zur herkömmlichen zephalometrischen Röntgenaufnahme den Vorteil, dass Strukturen zwischen den Segment-Volumenbereichen nicht projiziert werden und damit im Kompositbild nicht abgebildet werden.

Vorteilhafterweise können die Bilddaten der Segment-Volumenbereiche mittels der MRT-Vorrichtung aufgenommen werden, indem eine einzelne dreidimensionale MRT-Gesamtaufnahme des dentalen Objekts mit dem Objektvolumen aufgenommen wird und die Bilddaten der einzelnen Segment-Volumenbereiche aus der dreidimensionalen MRT-Gesamtaufnahme übernommen werden.

Bei dieser Ausführungsform wird die MRT-Gesamtaufnahme, beispielsweise des gesamten Schädels des Patienten aufgenommen, wobei der erste Segment-Volumenbereich mit der Midsagittalebene, der zweite Segment-Volumenbereich mit der linken Seite des Kiefers und der dritte Segmentvolumenbereich mit der rechten Seite des Kiefers aus der MRT-Gesamtaufnahme ausgeschnitten und übernommen werden.

Vorteilhafterweise können die Bilddaten der Segment-Volumenbereiche mittels der MRT-Vorrichtung aufgenommen werden, indem mehrere MRT-Segmentaufnahmen mittels der MRT-Vorrichtung aufgenommen werden, die die festgelegten Segment-Volumenbereiche abbilden.

Bei dieser alternativen Ausführungsform werden die einzelnen Segment-Volumenbereiche einzeln vermessen, wobei zu jedem Segment-Volumenbereich eine MRT-Segmentaufnahme erzeugt wird. Dabei wird die MRT-Vorrichtung so eingestellt, dass nur ein Volumen innerhalb des jeweiligen Segment-Volumenbereichs erfasst wird und in der MRT-Segmentaufnahme abgebildet wird.

Vorteilhafterweise können die einzelnen MRT-Segmentaufnahmen schrittweise nacheinander mittels der MRT-Vorrichtung vermessen werden.

Für jede MRT-Segmentaufnahme wird die MRT-Vorrichtung also neu eingestellt, um den jeweiligen Segment-Volumenbereich zu vermessen. Die einzelnen Schichten der MRT-Segmentaufnahmen werden also nacheinander ausgelesen, beispielsweise indem der Phasenkodiergradient zur Ortskodierung entsprechend eingestellt wird.

Vorteilhafterweise können zumindest zwei MRT-Segmentaufnahmen gleichzeitig mittels einer speziellen MRT-Vorrichtung vermessen werden, die auf einem Multi-Schicht-Anregungsverfahren beruht.

Die spezielle MRT-Vorrichtung kann also die MRT-Segmentaufnahmen gleichzeitig aufnehmen, wobei eine solche MRT-Vorrichtung die Anregung mehrerer Schichten im Objektvolumen ermöglicht und auf ein sogenanntes Multi-Puls-Anregungs-Verfahren beruht, wie im folgenden Fachartikel beschrieben (Benedikt A. Poser. Simultaneous multi-slice excitation by parallel transmission. Magn Reson Med. 2014 April; 71 (4): 1416-1427).

Mehrere Segment-Volumenbereiche können auch gleichzeitig bzw. interleaved aufgenommen werden, indem Anregungssignale und Auslesesignale gemischt werden.

Vorteilhafterweise kann jede MRT-Segmentaufnahme aus einer einzelnen MRT-Schichtaufnahme bestehen oder aus einem Stapel mehrerer MRT-Schichtaufnahmen bestehen.

Dadurch kann also die MRT-Segmentaufnahme aus einem einzelnen Stapel mehrerer MRT-Schichtaufnahmen gebildet sein, so dass die MRT-Segmentaufnahme entweder in einem Schritt mittels der MRT-Vorrichtung oder in mehreren nacheinander folgenden Schritten aufgenommen wird.

Vorteilhafterweise können die MRT-Schichtaufnahmen eines Stapels innerhalb des jeweiligen Segment-Volumenbereichs parallel zueinander angeordnet sein.

Dadurch wird jede der MRT-Schichtaufnahmen Schicht für Schicht nacheinander vermessen.

Vorteilhafterweise kann die Midsagittalebene des Schädels festgelegt werden, indem der Patient mittels einer Positionierungsvorrichtung relativ zur MRT-Vorrichtung positioniert wird oder indem eine Voraufnahme des Schädels des Patienten durchgeführt wird und anhand der Voraufnahme die Midsagittalebene und/oder die Lage und Orientierung der einzelnen Segment-Volumenbereiche manuell durch einen Benutzer oder automatisch mittels eines Computers bestimmt wird.

Dadurch wird die Midsagittalebene und/oder die Lage und Orientierung der einzelnen Segment-Volumenbereiche mittels der Positionierungsvorrichtung oder mittels der Voraufnahme bestimmt, so dass die Zielebene parallel zur Midsagittalebene und die Anordnung des ersten Segment-Volumenbereichs umfassend die Midsagittalebene in Abhängigkeit von der Lage der bestimmten Midsagittalebene festgelegt werden können.

Vorteilhafterweise kann das Kompositbild aus den Bilddaten der einzelnen Segment-Volumenbereiche computergestützt durch Projektion der Bilddaten auf die Zielebene erzeugt werden, indem die Bilddaten der einzelnen Segment-Volumenbereiche in einer gemeinsamen Projektionsrichtung auf die Zielebene projiziert werden, wobei die Projektionsrichtung senkrecht zur Zielebene angeordnet ist.

Dadurch werden also die Bilddaten der Segment-Volumenbereiche senkrecht zur Zielebene und damit senkrecht zur Midsagittalebene entlang der Projektionsrichtung projiziert. Idealerweise ist das linke Kiefergelenk und das rechte Kiefergelenk in Überlagerung im Kompositbild dargestellt. Ein solches Kompositbild ermöglicht eine genaue zephalometrische Diagnose des Patienten, da die Abstände zwischen den maßgeblichen anatomischen Strukturen genau vermessen werden können.

Vorteilhafterweise kann vor der Erfassung der einzelnen Segment-Volumenbereiche im Objektvolumen, die Lage und/oder die Ausrichtung der Segment-Volumenbereiche relativ zur MRT-Vorrichtung durch einen Benutzer festgelegt werden, wobei ein Musterkopf mit einem Muster-Oberkiefer und/oder einem Muster-Unterkiefer schematisch mittels einer Anzeigevorrichtung dargestellt wird, wobei die Segment-Volumenbereiche und deren Anordnung relativ zum Musterkopf graphisch dargestellt werden.

Dadurch wird dem Benutzer graphisch deutlich gemacht, an welchen Stellen die Segment-Volumenbereiche und die festgelegte Zielebene relativ zum Musterkopf liegen. Dadurch wird es dem Benutzer erleichtert, das erzeugte Kompositbild zu beurteilen.

Vorteilhafterweise kann das erzeugte Kompositbild mittels einer Anzeigevorrichtung dargestellt werden.

Dadurch kann das Kompositbild mittels der Anzeigevorrichtung, wie eines Monitors, so dass einem Benutzer die Diagnose des Kompositbildes erleichtert wird.

Vorteilhafterweise können zusätzlich zum Kompositbild die Bilddaten mindestens eines Segment-Volumenbereichs mittels der Anzeigevorrichtung dargestellt werden.

Dadurch kann der Benutzer auch die Bilddaten der Segment-Volumenbereiche zur Diagnose heranziehen. Denn im zweiten Segment-Volumenbereich, beispielsweise nur die linke Seite der Kiefer dargestellt, wobei im dritten Segment-Volumenbereich nur die rechte Seite der Kiefer dargestellt ist.

Die Bilddaten der Segment-Volumenbereiche können dreidimensionale Bilddaten oder zweidimensionale Bilddaten sein. Aufgenommen wird also immer ein dreidimensionaler Bereich des Objektvolumens also ein Voxel des jeweiligen Segment-Volumenbereichs. Zweidimensionale Bilddaten haben also entlang einer der Achsen des jeweiligen Segment-Volumenbereichs nur eine Schicht Voxel, während die dreidimensionalen Bilddaten entlang aller Achsen des jeweiligen Segment-Volumenbereichs mehrere Voxel aufweisen. Die Projektion erfolgt jedoch in beiden Fällen auf die gleiche Art und Weise, nämlich indem die vorhandenen Voxel (eine Schicht oder mehrerer Schichten) des Segment-Volumenbereichs entlang einer Projektionsrichtung, die senkrecht zur Zielebene angeordnet sein kann, auf die Zielebene projiziert und aggregiert werden. Es können also Voxel aus unterschiedlichen Schichten des jeweiligen Segment-Volumenbereichs auf die Zielebene projiziert werden.

### Kurzbeschreibung der Zeichnungen

Die Erfindung wird anhand der Zeichnungen erläutert. Es zeigt, die
- Fig. 1: eine Skizze zur Verdeutlichung des vorliegenden Verfahrens, die
- Fig. 2: ein schematisches Ablaufdiagramm, die
- Fig. 3: eine Skizze mit mehreren Kompositbildern.

### Ausführungsbeispiele

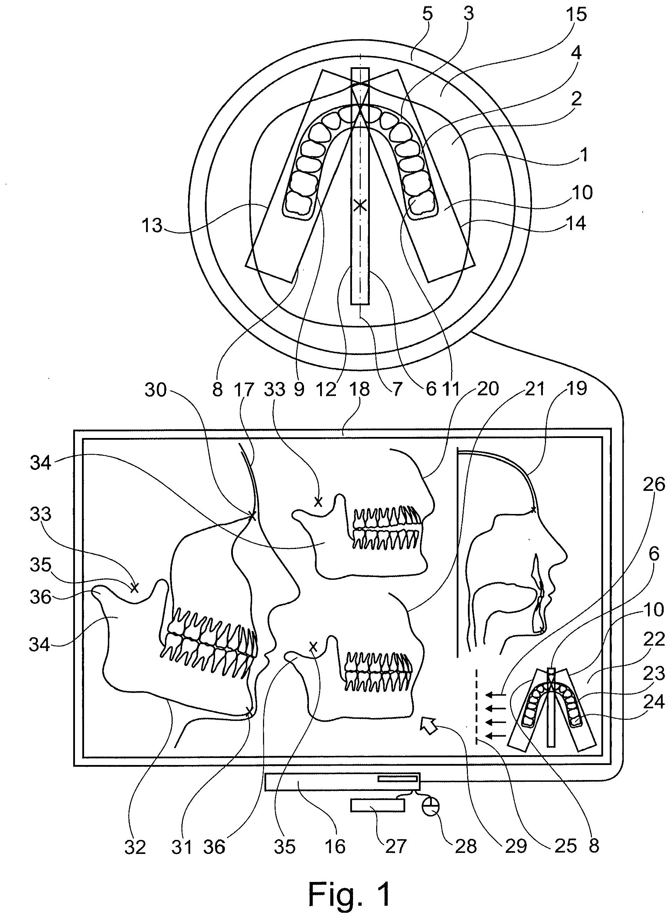

Die Fig. 1 zeigt eine Skizze zur Verdeutlichung des vorliegenden Verfahrens zur Erfassung eines dentalen Objekts 1, insbesondere zumindest eines Teils eines Schädels 2, eines Oberkiefers 3 und/oder eines Unterkiefers 4 mittels einer MRT-Vorrichtung 5. Dabei wird ein erster Segment-Volumenbereich festgelegt, der eine Midsagittalebene 7 zumindest teilweise umfasst, die durch die Strichpunktlinie dargestellt ist. Ein zweiter Segment-Volumenbereich 8 umfasst eine linke Seite 9 des Oberkiefers 3 und/oder des Unterkiefers 4. Ein dritter Segment-Volumenbereich 10 umfasst eine rechte Seite 11 des Oberkiefers 3 und/oder des Unterkiefers 4. Die Segment-Volumenbereiche 6, 8 und 10 sind quaderförmig ausgeführt und weisen eine Vorzugsrichtung auf, die durch die längste Kante des quaderförmigen Segment-Volumenbereichs definiert ist. Eine erste Vorzugsrichtung 12 des ersten Segment-Volumenbereichs 6, eine zweite Vorzugsrichtung 13 des zweiten Segment-Volumenbereichs 8 und eine dritte Vorzugsrichtung 14 des dritten Segment-Volumenbereichs 10 sind nicht parallel zueinander. Die Segment-Volumenbereiche 6, 8 und 10 überlagern sich nur teilweise. Bei einer ersten Ausführungsform werden die Bilddaten der Segment-Volumenbereiche 6, 8 und 10 aus einer MRT-Gesamtaufnahme des ganzen Kopfes 1 ausgeschnitten. Bei einer zweiten Ausführungsform werden die Bilddaten der einzelnen Segment-Volumenbereiche 6, 8 und 10 erzeugt, in dem einzelne MRT-Segmentaufnahmen der jeweiligen Segment-Volumenbereiche 6, 8 und 10 durchgeführt werden. Die innerhalb eines Messvolumens 15, der MRT-Vorrichtung aufgenommen Bilddaten werden von der MRT-Vorrichtung 5 an einen Computer 16 übermittelt. Aus den Bilddaten der einzelnen Segment-Volumenbereiche 6, 8 und 10 wird mittels des Computers 16 ein zweidimensionales Kompositbild 17 durch Projektion der Bilddaten auf eine Zielebene erzeugt, wobei die Zielebene parallel zu der festgelegten Midsagittalebene 7 angeordnet ist. Die Midsagittalebene 7 des Schädels zwei kann festgelegt werden, in dem der Patient mittels einer Positionierungsvorrichtung, wie einer Kopfhalterung oder einer Aufbisshalterung relativ zur MRT-Vorrichtung 5 positioniert wird oder in dem eine Voraufnahme des Patienten analysiert wird. Das erzeugte Kompositbild 17 kann mittels einer Anzeigevorrichtung 18, wie eines Monitors, graphisch dargestellt werden. Zusätzlich zum Kompositbild 17 können auch die ersten Bilddaten 19 des ersten Segmentvolumens 6, die zweiten Bilddaten 20 des zweiten Segment-Volumens 8 und die dritten Bilddaten 21 des dritten Segment-Volumens 10 graphisch dargestellt werden. In einer schematischen Darstellung 22 kann ein Musterkopf mit einem Musteroberkiefer 23 und/oder einem Muster-Unterkiefer 24 zusammen mit den festgelegten Segment-Volumenbereichen 6, 8 und 10 graphisch dargestellt werden. Die Anordnung der Zielebene 25 relativ zu den festgelegten Segment-Volumenbereichen 6, 8 und 10 sowie eine Projektionsrichtung 26 können ebenfalls graphisch dargestellt werden. Die schematische Darstellung 22 erleichtert dem Benutzer die Diagnose des Kompositbildes 17 und der Bilddaten 19, 20 und 21. An den Computer 16 sind Eingabemittel, wie eine Tastatur 27 und eine Maus 28 angeschlossen, die die Bedienung eines virtuellen Werkzeugs über einen Cursor 29 ermöglichen, um beispielsweise die Lage und Ausrichtung der einzelnen Segment-Volumenbereiche 6, 8 und 10 oder die Lage der Midsagittalebene 7 oder der Zielebene 25 manuell festzulegen. Das Kompositbild 17 entspricht einer herkömmlichen zephalometrischen Röntgenaufnahme zur Durchführung einer zephalometrischen Diagnose. Im Kompositbild 17 sind wesentliche anatomische Strukturen, wie ein Punkt 30 der vorderen Schädelbasis, ein Punkt 31 des knöchelnden Kinns 32, eine linke Kiefergelenksachse 33 des linken Kiefergelenks 34, eine rechte Kiefergelenksachse 35 des rechten Kiefergelenks 36, dargestellt. Im vorliegenden Kompositbild 17 stimmt die linke Kiefergelenksachse 33 mit der rechten Kiefergelenksachse überein. Bei der zephalometrischen Diagnose können die Abstände zwischen den genannten Punkten 30, 31, 33 und 35 ermittelt werden.

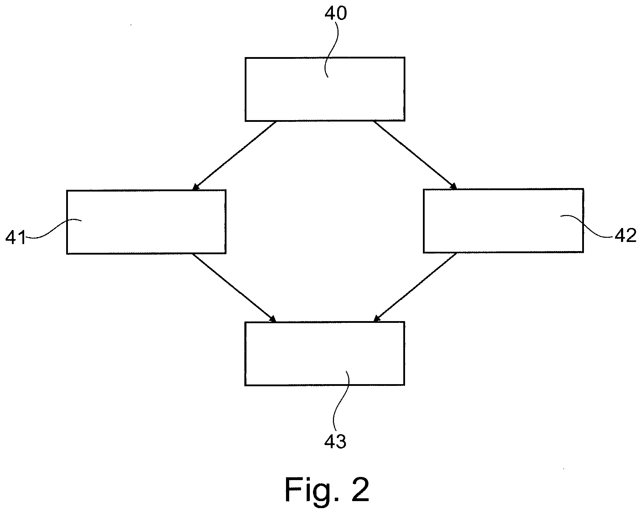

Die Fig. 2 zeigt ein schematisches Ablaufdiagramm zur Erläuterung einer Ausführungsform des vorliegenden Verfahrens. Bei einem ersten Schritt 40 werden die Segment-Volumenbereiche 6, 8 und 10 relativ zur MRT-Vorrichtung festgelegt. Bei einer ersten Alternative des zweiten Schritts 41 werden die Bilddaten der Segment-Volumenbereiche 6, 8 und 10 mittels der MRT-Vorrichtung 5 aufgenommen, in dem eine MRT-Gesamtaufnahme des Patientenkopfes 1 aufgenommen wird und die Bilddaten der Segment-Volumenbereiche 6, 8 und 10 aus der MRT-Gesamtaufnahme ausgeschnitten werden. Bei einer zweiten Ausführungsform des zweiten Schritts 42 werden die Bilddaten der Segment-Volumenbereiche 6, 8 und 10 aufgenommen, in dem einzelne MRT-Segmentaufnahmen mittels der MRT-Vorrichtung 5 nacheinander vermessen werden. Bei einem dritten Schritt 43 werden die Bilddaten 19, 20 und 21 der Segment-Bereiche 6, 8 und 10 mittels des Computers 16 durch Projektion auf die Zielebene 25 abgebildet und das Kompositbild 17 erzeugt.

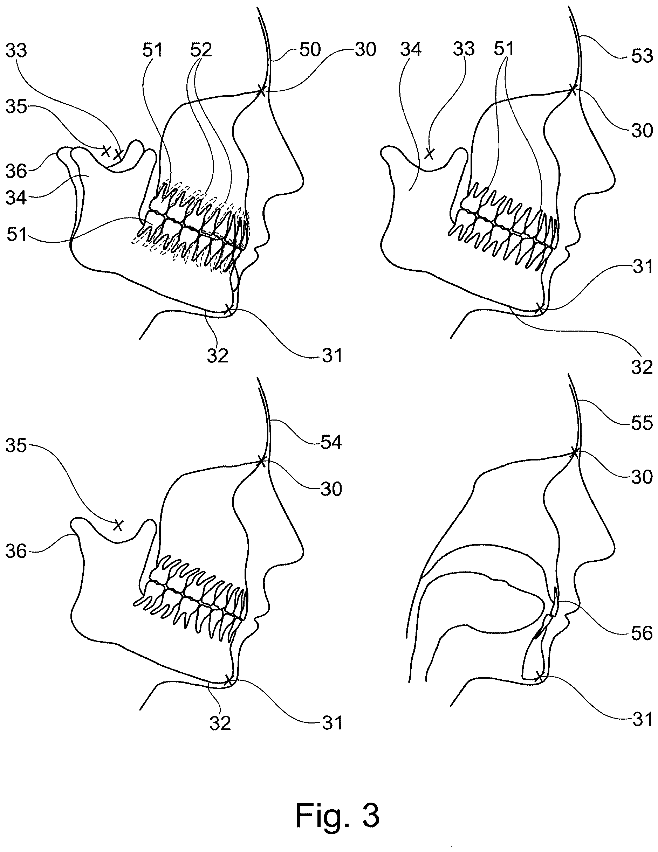

Die Fig. 3 zeigt eine Skizze unterschiedlicher Kompositbilder. Das erste Kompositbild 50 ist, wie in Fig. 1, durch Projektion der ersten Bilddaten 19 des ersten Segment-Volumens 6, der zweiten Bilddaten 20 des zweiten Segment-Volumens 8 und der dritten Bilddaten 21 des dritten Segment-Volumens 10 auf die Zielebene 25 erzeugt worden.

Im Unterschied zu Fig. 1 weist der Patient eine bifaciale Asymmetrie auf, so dass die linke Kiefergelenksachse 33 des linken Kiefergelenks 34 von der rechten Kiefergelenkachse 35 des rechten Kiefergelenks 36 im Kompositbild 50 abweicht. Die Zähne 51 der linken Kieferseite 9, die durch volle Linien dargestellt sind, unterscheiden sich auch deutlich von den Zähne 52 der rechten Kieferseite 11, die durch gestrichelte Linien dargestellt sind.

Das zweite Kompositbild 53 ist durch Projektion der ersten Bilddaten 19 des ersten Segment-Volumens 6 und der zweiten Bilddaten 20 des zweiten Segment-Volumens 8 der linken Kieferseite 9 auf die Zielebene 25 erzeugt worden. Dadurch sind also lediglich das linke Kiefergelenk 34 und die Zähne 51 der linken Kieferseite 9 dargestellt.

Das dritte Kompositbild 54 ist durch Projektion der ersten Bilddaten 19 des ersten Segment-Volumens 6 und der dritten Bilddaten 21 des dritten Segment-Volumens 10 der rechten Kieferseite 11 auf die Zielebene 25 erzeugt worden. Dadurch sind also lediglich das rechte Kiefergelenk 36 und die Zähne 52 der rechten Kieferseite 11 dargestellt.

Das vierte Kompositbild 55 ist durch Projektion der ersten Bilddaten 19 des ersten Segment-Volumens 6 auf die Zielebene 25 erzeugt worden. Dadurch sind also lediglich die anatomischen Strukturen 30,31 und zumindest teilweise Schneidezähne 56 aus dem ersten Segment-Volumenbereich 6 mit der Midsagittalebene 7 sichtbar.

Die einzelnen Kompositbilder 50, 53, 54 und 55 können mittels der Anzeigevorrichtung 18 gleichzeitig nebeneinander oder auch nacheinander dargestellt werden, um dem Benutzer eine bessere Diagnose zu ermöglichen.

### Bezugszeichen

- 1: Kopf
- 2: Schädel
- 3: Oberkiefer
- 4: Unterkiefer
- 5: MRT-Vorrichtung
- 6: erster Segment-Volumenbereich
- 7: Midsagittalebene
- 8: zweiter Segment-Volumenbereich
- 9: linke Seite des Kiefers
- 10: dritter Segment-Volumenbereich
- 11: rechte Seite des Kiefers
- 12: erste Vorzugsrichtung
- 13: zweite Vorzugsrichtung
- 14: dritte Vorzugsrichtung
- 15: Messvolumen
- 16: Computer
- 17: Kompositbild
- 18: Anzeigevorrichtung
- 19: erste Bilddaten
- 20: zweite Bilddaten
- 21: dritte Bilddaten
- 22: schematische Darstellung
- 23: Musteroberkiefer
- 24: Muster-Unterkiefer
- 25: Zielebene
- 26: Projektionsrichtung
- 27: Tastatur
- 28: Maus
- 29: Cursor
- 30: Punkt der vorderen Schädelbasis
- 31: Punkt des knöchelnden Kinns
- 32: Kinn
- 33: linke Kiefergelenkachse
- 34: linkes Kiefergelenk
- 35: Kiefergelenkachse
- 36: rechtes Kiefergelenk
- 40: erster Schritt
- 41: zweiter Schritt
- 42: zweiter Schritt
- 43: dritte Schritt
- 50: erstes Kompositbild
- 51: Zähne der linken Kieferseite
- 52: Zähne der rechten Kieferseite
- 53: zweites Kompositbild
- 54: drittes Kompositbild
- 55: viertes Kompositbild
- 56: Schneidezähne

## Patentansprüche

1. Verfahren zur Erfassung eines dentalen Objekts (1) eines Patienten mit einem Objektvolumen, insbesondere zumindest eines Teils eines Schädels (2), eines Oberkiefers (3) und/oder eines Unterkiefers (4), **dadurch gekennzeichnet, dass** mehrere Segment-Volumenbereiche (6, 8, 10) im Objektvolumen festgelegt werden, wobei die Segment-Volumenbereiche (6, 8, 10) sich höchstens teilweise überlagern und Vorzugsrichtungen (12, 13, 14) der Segment-Volumenbereiche (6, 8, 10) nicht parallel zueinander sind, wobei Bilddaten (19, 20 ,21) der Segment-Volumenbereiche (6, 8, 10) mittels einer MRT-Vorrichtung (5) innerhalb eines Meßvolumens (15) der MRT-Vorrichtung (5) aufgenommen werden, wobei aus den Bilddaten (19, 20 ,21) der einzelnen Segment-Volumenbereiche (6, 8, 10) computergestützt ein zweidimensionales Kompositbild (17) durch Projektion der Bilddaten (19, 20 ,21) auf eine Zielebene (25) erzeugt wird, wobei die Zielebene (25) parallel zu einer festgelegten Midsagittalebene (7) des Schädels (2) angeordnet ist oder der Midsagittalebene (7) entspricht, wobei ein erster Segment-Volumenbereich zumindest teilweise die Midsagittalebene (7) des Schädels umfasst.

2. Verfahren nach Anspruch 1, **dadurch gekennzeichnet, dass** ein zweiter Segment-Volumenbereich (8) festgelegt wird, der zumindest einen Teil des Oberkiefers (2) und/oder des Unterkiefers (3) umfasst, wobei aus dem ersten Segment-Volumenbereich (6) und dem zweiten Segment-Volumenbereich (8) durch Projektion (26) auf die Zielebene (25) das zweidimensionale Kompositbild (17) erzeugt wird.

3. Verfahren nach Anspruch 2, **dadurch gekennzeichnet, dass** der zweite Segment-Volumenbereich (8) eine linke Seite (9) des Oberkiefers (2) und/oder des Unterkiefers (3) umfasst, wobei zusätzlich ein dritter Segment-Volumenbereich (10) festgelegt wird, der eine rechte Seite (11) des Oberkiefers (2) und/oder des Unterkiefers (3) umfasst, wobei aus dem ersten Segment-Volumenbereich (6), dem zweiten Segment-Volumenbereich (8) und dem dritten Segment-Volumenbereich (10) durch Projektion auf die Zielebene (25) das zweidimensionale Kompositbild (17) erzeugt wird.

4. Verfahren nach einem der Ansprüche 1 bis 3, **dadurch gekennzeichnet, dass** jeder Segment-Volumenbereich (6, 8, 10) bezüglich seiner Lage und Ausrichtung innerhalb des Meßvolumens (15) der MRT-Vorrichtung (5) anhand einer Voreinstellung für den jeweiligen Patienten oder anhand einer Voraufnahme des Objekts (1) festgelegt wird.

5. Verfahren nach einem der Ansprüche 1 bis 4, **dadurch gekennzeichnet, dass** eine Tiefe des Segment-Volumenbereichs (6, 8, 10) zwischen 0,5 mm und 30 mm, vorzugsweise zwischen 1 mm und 15 mm, beträgt.

6. Verfahren nach einem der Ansprüche 1 bis 5, **dadurch gekennzeichnet, dass** das Kompositbild (17) einer herkömmlichen kephalometrischen Röntgenaufnahme entspricht, wobei die Zielebene (25) des Kompositbildes (17) einer Ebene entspricht, die parallel zu einer Detektorfläche eines Röntgendetektors bei einer kephalometrischen Röntgenaufnahme angeordnet ist.

7. Verfahren nach einem der Ansprüche 1 bis 6, **dadurch gekennzeichnet, dass** die Bilddaten (19, 20 ,21) der Segment-Volumenbereiche (6, 8, 10) mittels der MRT-Vorrichtung (5) aufgenommen werden, indem eine einzelne dreidimensionale MRT-Gesamtaufnahme des dentalen Objekts mit dem Objektvolumen aufgenommen wird und die Bilddaten (19, 20 ,21) der einzelnen Segment-Volumenbereiche (6, 8, 10) aus der dreidimensionalen MRT-Gesamtaufnahme übernommen werden.

8. Verfahren nach einem der Ansprüche 1 bis 6, **dadurch gekennzeichnet, dass** die Bilddaten (19, 20 ,21) der Segment-Volumenbereiche (6, 8, 10) mittels der MRT-Vorrichtung (5) aufgenommen werden, indem mehrere MRT-Segmentaufnahmen mittels der MRT-Vorrichtung (5) aufgenommen werden, die die festgelegte Segment-Volumenbereiche (6, 8, 10) abbilden.

9. Verfahren nach Anspruch 8, **dadurch gekennzeichnet, dass** die einzelnen MRT-Segmentaufnahmen schrittweise nacheinander mittels der MRT-Vorrichtung (5) vermessen werden.

10. Verfahren nach Anspruch 8 oder 9, **dadurch gekennzeichnet, dass** zumindest zwei MRT-Segmentaufnahmen gleichzeitig mittels einer speziellen MRT-Vorrichtung vermessen werden, die auf einem Multi-Schicht-Anregungsverfahren beruht.

11. Verfahren nach einem der Ansprüche 8 bis 10, **dadurch gekennzeichnet, dass** jede MRT-Segmentaufnahme aus einer einzelnen MRT-Schichtaufnahme besteht oder aus einem Stapel mehrerer MRT-Schichtaufnahmen besteht, wobei die MRT-Schichtaufnahmen eines Stapels innerhalb des jeweiligen Segment-Volumenbereichs parallel zueinander angeordnet sind.

12. Verfahren nach einem der Ansprüche 1 bis 11, **dadurch gekennzeichnet, dass** die Midsagittalebene (7) und/oder die Lage und Orientierung der einzelnen Segment-Volumenbereiche (6, 8, 10) des Schädels (2) festgelegt werden, indem der Patient mittels einer Positionierungsvorrichtung relativ zur MRT-Vorrichtung (5) positioniert wird oder indem eine Voraufnahme des Schädels (2) des Patienten durchgeführt wird und anhand der Voraufnahme die Midsagittalebene (7) manuell durch einen Benutzer oder automatisch mittels eines Computers (16) bestimmt wird.

13. Verfahren nach einem der Ansprüche 1 bis 12, **dadurch gekennzeichnet, dass** das Kompositbild (17) aus den Bilddaten (19, 20 ,21) der einzelnen Segment-Volumenbereiche (6, 8, 10) computergestützt durch Projektion der Bilddaten (19, 20 ,21) auf die Zielebene (25) erzeugt wird, indem die Bilddaten (19, 20 ,21) der einzelnen Segment-Volumenbereiche (6, 8, 10) in einer gemeinsamen Projektionsrichtung (26) auf die Zielebene (25) projiziert werden, wobei die Projektionsrichtung (26) senkrecht zur Zielebene (25) angeordnet ist.

14. Verfahren nach einem der Ansprüche 1 bis 13, **dadurch gekennzeichnet, dass** vor der Erfassung der einzelnen Segment-Volumenbereiche (6, 8, 10) im Objektvolumen, die Lage und/oder die Ausrichtung der Segment-Volumenbereiche (6, 8, 10) relativ zur MRT-Vorrichtung (5) durch einen Benutzer festgelegt werden, wobei ein Musterkopf mit einem Muster-Oberkiefer (23) und/oder einem Muster-Unterkiefer (24) schematisch mittels einer Anzeigevorrichtung (18) dargestellt wird, wobei die Segment-Volumenbereiche (6, 8, 10) und deren Anordnung relativ zum Musterkopf und/oder die Zielebene (25) und/oder die Midsagittalebene (7) graphisch dargestellt werden.

15. Verfahren nach einem der Ansprüche 1 bis 14, **dadurch gekennzeichnet, dass** das erzeugte Kompositbild (17) mittels einer Anzeigevorrichtung (18) dargestellt wird, wobei zusätzlich zum Kompositbild (17) die Bilddaten (19, 20 ,21) mindestens eines Segment-Volumenbereichs (6, 8, 10) mittels der Anzeigevorrichtung (18) dargestellt werden.

## Claims

1. Method for acquiring a dental object (1) of a patient with an object volume, in particular at least a part of a skull (2), an upper jaw (3) and/or a lower jaw (4), **characterized in that** a plurality of segment volume ranges (6, 8, 10) are defined in the object volume, wherein the segment volume ranges (6, 8, 10) overlap at most partially, and preferred directions (12, 13, 14) of the segment volume ranges (6, 8, 10) are not parallel to one another, wherein image data (19, 20, 21) of the segment volume ranges (6, 8, 10) is recorded by means of an MRI machine (5) within a measuring volume (15) of the MRI machine (5), wherein, computer-assisted, a two-dimensional composite image (17) is generated from the image data (19, 20, 21) of the individual segment volume ranges (6, 8, 10) by projecting the image data (19, 20, 21) onto a target plane (25), wherein the target plane (25) is disposed parallel to a defined midsagittal plane (7) of the skull (2) or corresponds to the midsagittal plane (7), wherein a first segment volume range at least partially comprises the midsagittal plane (7) of the skull.

2. Method according to Claim 1, **characterized in that** a second segment volume range (8) is defined, which comprises at least a part of the upper jaw (2) and/or the lower jaw (3), wherein the two-dimensional composite image (17) is generated from the first segment volume range (6) and the second segment volume range (8) by projection (26) onto the target plane (25).

3. Method according to Claim 2, **characterized in that** the second segment volume range (8) comprises a left side (9) of the upper jaw (2) and/or the lower jaw (3), wherein additionally a third segment volume range (10) is defined, which comprises a right side (11) of the upper jaw (2) and/or the lower jaw (3), wherein the two-dimensional composite image (17) is generated from the first segment volume range (6), the second segment volume range (8) and the third segment volume range (10) by projection onto the target plane (25) .

4. Method according to any of Claims 1 to 3, **characterized in that**, with respect to its position and orientation within the measuring volume (15) of the MRI machine (5), each segment volume range (6, 8, 10) is defined on the basis of a presetting for the respective patient or on the basis of a preliminary image of the object (1).

5. Method according to any of Claims 1 to 4, **characterized in that** a depth of the segment volume range (6, 8, 10) is between 0.5 mm and 30 mm, preferably between 1 mm and 15 mm.

6. Method according to any of Claims 1 to 5, **characterized in that** the composite image (17) corresponds to a conventional cephalometric radiograph, wherein the target plane (25) of the composite image (17) corresponds to a plane, which is disposed parallel to a detector surface of an X-ray detector for a cephalometric radiograph.

7. Method according to any of Claims 1 to 6, **characterized in that** the image data (19, 20, 21) of the segment volume ranges (6, 8, 10) is recorded by means of the MRI machine (5), **in that** a single three-dimensional MRI overall image of the dental object is recorded with the object volume, and the image data (19, 20, 21) of the individual segment volume ranges (6, 8, 10) is carried over from the three-dimensional MRI overall image.

8. Method according to any of Claims 1 to 6, **characterized in that** the image data (19, 20, 21) of the segment volume ranges (6, 8, 10) is recorded by means of the MRI machine (5), **in that** a plurality of MRI segment acquisitions, which display the defined segment volume ranges (6, 8, 10), are recorded by means of the MRI machine (5).

9. Method according to Claim 8, **characterized in that** the individual MRI segment acquisitions are incrementally measured by means of the MRI machine (5).

10. Method according to Claim 8 or 9, **characterized in that** at least two MRI segment acquisitions are measured at the same time by means of a special MRI machine, which is based on a multislice excitation method.

11. Method according to any of Claims 8 to 10, **characterized in that** each MRI segment acquisition consists of a single MRI slice image or a stack of multiple MRI slice images, wherein the MRI slice images of a stack are arranged parallel to one another within the respective segment volume range.

12. Method according to any of Claims 1 to 11, **characterized in that** the midsagittal plane (7) and/or the position and orientation of the individual segment volume ranges (6, 8, 10) of the skull (2) are defined **in that** the patient is positioned relative to the MRI machine (5) by means of a positioning device, or **in that** a preliminary image of the skull (2) of the patient is taken and the midsagittal plane (7) is determined manually by a user or automatically by means of a computer (16) on the basis of the preliminary image.

13. Method according to any of Claims 1 to 12, **characterized in that** the composite image (17) is generated in a computer-assisted manner from the image data (19, 20, 21) of the individual segment volume ranges (6, 8, 10) by projection of the image data (19, 20, 21) onto the target plane (25), **in that** the image data (19, 20, 21) of the individual segment volume ranges (6, 8, 10) is projected onto the target plane (25) in a common projection direction (26), wherein the projection direction (26) is arranged perpendicular to the target plane (25).

14. Method according to any of Claims 1 to 13, **characterized in that**, prior to the acquisition of the individual segment volume ranges (6, 8, 10) in the object volume, the position and/or the orientation of the segment volume ranges (6, 8, 10) in relation to the MRI machine (5) are defined by a user, wherein a head template with an upper jaw template (23) and/or a lower jaw template (24) is schematically displayed by means of a display device (18), wherein the segment volume ranges (6, 8, 10) and the arrangement thereof in relation to the head template and/or the target plane (25) and/or the midsagittal plane (7) are graphically displayed.

15. Method according to any of Claims 1 to 14, **characterized in that** the generated composite image (17) is displayed by means of a display device (18), wherein, in addition to the composite image (17), the image data (19, 20, 21) of at least one segment volume range (6, 8, 10) is displayed by means of the display device (18).

## Revendications

1. Procédé pour la détection d'un objet dentaire(1) d'un patient ayant un volume d'objet, en particulier au moins une partie d'un crane (2), d'un maxillaire supérieur (3) et/ou d'un maxillaire inférieur (4), **caractérisé en ce que** plusieurs zones de volume de segment (6, 8, 10) sont définies dans le volume d'objet, les zones de volume de segment (6, 8, 10) se chevauchant au plus partiellement et des directions préférentielles (12, 13, 14) des zones de volume de segment (6, 8, 10) n'étant pas parallèles les unes aux autres, des données d'image (19, 20, 21) des zones de volume de segment (6, 8, 10) étant enregistrées au moyen d'un dispositif IRM (5) à l'intérieur d'un volume de mesure (15) du dispositif IRM (5), une image composite bidimensionnelle (17) étant générée par ordinateur à partir des données d'image (19, 20, 21) des zones de volume de segment (6, 8, 10) individuelles par la projection des données d'image (19, 20, 21) sur un plan cible (25), le plan cible (25) étant disposé parallèlement à un plan sagittal médian (7) défini du crâne (2) ou correspondant au plan sagittal médian (7), une première zone de volume de segment comprenant au moins partialement le plan sagittal médian (7).

2. Procédé selon la revendication 1, **caractérisé en ce qu'**une deuxième zone de volume de segment (8) est définie, laquelle comprend au moins une partie du maxillaire supérieur (2) et/ou du maxillaire inférieur (3), l'image composite bidimensionnelle (17) étant générée à partir de la première zone de volume de segment (6) et de la deuxième zone de volume de segment (8) par projection (26) sur le plan cible (25) .

3. Procédé selon la revendication 2, **caractérisé en ce que** la deuxième zone de volume de segment (8) comprend un côté gauche (9) du maxillaire supérieur (2) et/ou du maxillaire inférieur (3), une troisième zone de volume de segment (10) étant en plus définie, laquelle comprend un côté droit (11) du maxillaire supérieur (2) et/ou du maxillaire inférieur (3), l'image composite bidimensionnelle (17) étant générée à partir à partir de la première zone de volume de segment (6), de la deuxième zone de volume de segment (8) et de la troisième zone de volume de segment (10) par projection sur le plan cible (25).

4. Procédé selon l'une quelconque des revendications 1 à 3, **caractérisé en ce que** chaque zone de volume de segment (6, 8, 10) est définie en relation avec sa position et son orientation à l'intérieur du volume de mesure (15) du dispositif IRM (5) au moyen d'un préréglage pour le patient respectif ou au moyen d'un pré-enregistrement de l'objet (1).

5. Procédé selon l'une quelconque des revendications 1 à 4, **caractérisé en ce que** la profondeur de la zone de volume de segment (6, 8, 10) est comprise entre 0,5 mm et 30 mm, de préférence entre 1 mm et 15 mm.

6. Procédé selon l'une quelconque des revendications 1 à 5, **caractérisé en ce que** l'image composite (17) correspond à une radiographie céphalométrique conventionnelle, le plan cible (25) de l'image composite (17) correspondant à un plan qui est disposé parallèlement à une surface de détecteur d'un détecteur de rayons X dans le cas d'une radiographie céphalométrique.

7. Procédé selon l'une quelconque des revendications 1 à 6, **caractérisé en ce que** les données d'image (19, 20, 21) des zones de volume de segment (6, 8, 10) sont enregistrées au moyen du dispositif IRM (5), en enregistrant un enregistrement IRM complet tridimensionnel individuel de l'objet dentaire et en reprenant les données d'image (19, 20, 21) des zones de volume de segment (6, 8, 10) individuelles de l'enregistrement IRM complet tridimensionnel.

8. Procédé selon l'une quelconque des revendications 1 à 6, **caractérisé en ce que** les données d'image (19, 20, 21) des zones de volume de segment (6, 8, 10) sont enregistrées au moyen du dispositif IRM (5), en enregistrant au moyen du dispositif IRM (5) plusieurs enregistrements IRM de segments qui représentent les zones de volume de segment (6, 8, 10) définies.

9. Procédé selon la revendication 8, **caractérisé en ce que** les enregistrements IRM de segments individuels sont mesurés successivement progressivement au moyen du dispositif IRM (5) .

10. Procédé selon la revendication 8 ou 9, **caractérisé en ce qu'**au moins deux enregistrements IRM de segments sont mesurés simultanément au moyen d'un dispositif IRM spécial, qui se base sur un procédé d'excitation multicouche.

11. Procédé selon l'une quelconque des revendications 8 à 10, **caractérisé en ce que** chaque enregistrement IRM de segment se compose d'un enregistrement IRM de couche individuel ou d'un empilement de plusieurs enregistrements IRM de couches, les enregistrements IRM de couches d'un empilement étant disposés en parallèle les uns aux autres à l'intérieur de la zone de volume de segment respective.

12. Procédé selon l'une quelconque des revendications 1 à 11, **caractérisé en ce que** le plan sagittal médian (7) et/ou la position et l'orientation des zones de volume de segment (6, 8, 10) individuelles du crane (2) sont définis par le positionnement du patient par rapport au dispositif IRM (5) au moyen d'un dispositif de positionnement ou par la réalisation d'un pré-enregistrement du crane (2) du patient et la détermination du plan sagittal médian (7) manuellement par un utilisateur ou automatiquement au moyen d'un ordinateur (16).

13. Procédé selon l'une quelconque des revendications 1 à 12, **caractérisé en ce que** l'image composite (17) est générée par ordinateur à partir des données d'image (19, 20, 21) des zones de volume de segment (6, 8, 10) individuelles par la projection des données d'image (19, 20, 21) sur le plan cible (25), en projetant les données d'image (19, 20, 21) des zones de volume de segment (6, 8, 10) individuelles dans une direction de projection (26) commune sur le plan cible (25), la direction de projection (26) étant disposée perpendiculairement au plan cible (25).

14. Procédé selon l'une quelconque des revendications 1 à 13, **caractérisé en ce qu'**avant la détection des zones de volume de segment (6, 8, 10) individuelles dans le volume d'objet, la position et/ou l'orientation des zones de volume de segment (6, 8, 10) par rapport au dispositif IRM (5) sont définies par un utilisateur, une tête modèle comprenant un maxillaire supérieur modèle (23) et/ou un maxillaire inférieur modale (24) étant représentée schématiquement au moyen d'un dispositif d'affichage (18), les zones de volume de segment (6, 8, 10) et leur disposition par rapport à la tête modèle et/ou le plan cible (25) et/ou le plan sagittal médian (7) étant représentés graphiquement.

15. Procédé selon l'une quelconque des revendications 1 à 14, **caractérisé en ce que** l'image composite (17) générée est représentée au moyen d'un dispositif d'affichage (18), les données d'image (19, 20, 21) d'au moins une zone de volume de segment (6, 8, 10) étant représentées en plus de l'image composite (17) au moyen du dispositif d'affichage (18).
